# EUROPEAN PATENT APPLICATION

(11) **EP 4 635 422 A1**
(43) Date of publication of application: **22.10.2025**
(21) Application number: 23903283.2
(22) Date of filing: 29.11.2023
(51) Int. Cl.: A61B 8/08

(54) **ULTRASONIC DIAGNOSTIC DEVICE AND METHOD FOR CONTROLLING ULTRASONIC DIAGNOSTIC DEVICE**

(30) Priority: 15.12.2022 JP 2022200197
(71) Applicant: FUJIFILM Corporation, Tokyo 106-8620 (JP)
(72) Inventor: KOSHINO Riko, Tokyo 106-8620 (JP)
(74) Representative: Klunker IP Patentanwälte PartG mbB
(86) International application number: PCT/JP2023/042710
(87) International publication number: WO 2024/127992

(57) **Abstract**

An ultrasonic diagnostic apparatus includes a first category determination unit (25) that determines, based on an ultrasonic image including a mammary gland region in a breast of a subject, a category of a glandular tissue component in the breast, and a category output unit (26) that outputs the category of the glandular tissue component determined by the first category determination unit (25).

## Description

### BACKGROUND OF THE INVENTION

### 1. Field of the Invention

The present invention relates to an ultrasonic diagnostic apparatus used for an examination of a breast of a subject and a method of controlling an ultrasonic diagnostic apparatus.

### 2. Description of the Related Art

In related art, an ultrasonic diagnostic apparatus using ultrasonic images is put into practical use in the medical field. In general, the ultrasonic diagnostic apparatus comprises an ultrasonic probe in which a transducer array is provided and an apparatus body connected to the ultrasonic probe, in which an ultrasonic beam is transmitted from the ultrasonic probe toward a subject, an ultrasonic echo from the subject is received by the ultrasonic probe, and a reception signal is electrically processed to generate the ultrasonic image.

A composition of a fat tissue and a mammary gland tissue in a breast varies depending on a person, while an anatomical structure of the breast is common, and the mammary gland tissue branches from a main duct to extralobular ducts, which then connect to numerous lobules. A stroma exists around the lobules, and the mammary gland tissue includes the stroma.

It is known that there are two types of stroma around the lobules, perilobular stroma and edematous stroma. The perilobular stroma exists along a structure from the lobule to the mammary duct, and includes many collagen fibers. On the other hand, the edematous stroma fills a space between the perilobular stroma, is rich in extracellular matrix, contains collagen fibers and fat that coexist, and fewer collagen fibers than the perilobular stroma.

In recent years, the concept of personalized risk management for patients becomes more widespread, and it is known that a ratio of a mammary gland region in the breast, particularly dense mammary gland, is a risk factor for cancer. The ratio of the mammary gland region in the breast can be measured by using a mammography apparatus.

In addition, in Su Hyun Lee et al., "Glandular Tissue Component and Breast Cancer Risk in Mammographically Dense Breasts at Screening Breast US", Radiology, Volume 301, October 1, 2021, it is reported that a cancer is likely to occur in a case in which a ratio of a glandular tissue component (GTC) region including mammary ducts, lobules, and perilobular stroma in the mammary gland region is high even though the mammary gland region is almost the same. That is, in addition to the ratio of the mammary gland region in the breast, a ratio of the GTC region in the mammary gland region may be a risk factor. This means a higher risk in a patient with less advanced atrophy of the lobule.

However, in the mammography apparatus, the perilobular stroma and the edematous stroma cannot be distinguished from each other, and the entire mammary gland tissue is observed as whitish, and as a result, the ratio of the GTC region in the mammary gland region cannot be measured.

JP2020-18694A discloses an ultrasonic diagnostic apparatus that extracts a mammary gland region by detecting a boundary in a depth direction of an ultrasonic image and detects a lesion part existing in the mammary gland region.

### SUMMARY OF THE INVENTION

However, an object of the ultrasonic diagnostic apparatus disclosed in JP2020-18694A is to detect the lesion part in the mammary gland region, and is not concerned with further subdividing the mammary gland region into smaller tissues. Therefore, there is a problem in that the risk of cancer in the mammary gland region cannot be considered in detail.

The present invention has been made in order to solve such a problem in the related art, and an object of the present invention is to provide an ultrasonic diagnostic apparatus that enables a user to consider a risk of cancer in a mammary gland region of a subject in detail.

With the following configurations, the above-described object can be achieved.
[1] An ultrasonic diagnostic apparatus comprising: a first category determination unit that determines, based on an ultrasonic image including a mammary gland region in a breast of a subject, a category of a glandular tissue component in the breast; and a category output unit that outputs the category of the glandular tissue component determined by the first category determination unit.
[2] The ultrasonic diagnostic apparatus according to [1], in which the first category determination unit determines the category of the glandular tissue component using a trained model that has been trained through machine learning based on a plurality of training data each including the ultrasonic image in which the breast is imaged and the category of the glandular tissue component in the breast.
[3] The ultrasonic diagnostic apparatus according to [1], in which the first category determination unit includes a mammary gland region extraction unit that extracts the mammary gland region from the ultrasonic image including the mammary gland region in the breast of the subject, and a second category determination unit that determines the category of the glandular tissue component in the breast based on the mammary gland region extracted by the mammary gland region extraction unit.
[4] The ultrasonic diagnostic apparatus according to [3], in which the second category determination unit determines the category of the glandular tissue component using a trained model that has been trained through machine learning based on a plurality of training data each including the ultrasonic image including the mammary gland region in the breast, the mammary gland region extracted by the mammary gland region extraction unit, and the category of the glandular tissue component in the breast.
[5] The ultrasonic diagnostic apparatus according to [3] or [4], in which the mammary gland region extraction unit extracts the mammary gland region by image-analyzing the ultrasonic image.
[6] The ultrasonic diagnostic apparatus according to [3] or [4], in which the mammary gland region extraction unit extracts the mammary gland region using a trained model that has been trained through machine learning based on a plurality of training data each including the ultrasonic image in which the breast is imaged and the mammary gland region in the breast.
[7] The ultrasonic diagnostic apparatus according to [1], in which the first category determination unit includes a mammary gland region extraction unit that extracts the mammary gland region from the ultrasonic image including the mammary gland region in the breast of the subject, a glandular tissue component region extraction unit that extracts a glandular tissue component region including a mammary duct, a lobule, and perilobular stroma in the mammary gland region, from the mammary gland region extracted by the mammary gland region extraction unit, and a third category determination unit that determines the category of the glandular tissue component in the breast based on the glandular tissue component region extracted by the glandular tissue component region extraction unit.
[8] The ultrasonic diagnostic apparatus according to [7], in which the third category determination unit determines the category of the glandular tissue component using a trained model that has been trained through machine learning based on a plurality of training data each including the ultrasonic image in which the glandular tissue component region is imaged and the category of the glandular tissue component in the breast.
[9] The ultrasonic diagnostic apparatus according to [7], in which the third category determination unit calculates a ratio of the glandular tissue component region to the mammary gland region, to determine the category of the glandular tissue component of the subject based on the calculated ratio of the glandular tissue component region to the mammary gland region.
[10] The ultrasonic diagnostic apparatus according to any one of [7] to [9], in which the glandular tissue component region extraction unit extracts the glandular tissue component region by image-analyzing the ultrasonic image in which the mammary gland region is imaged.
[11] The ultrasonic diagnostic apparatus according to any one of [7] to [10], in which the mammary gland region extraction unit extracts the mammary gland region by image-analyzing the ultrasonic image.
[12] The ultrasonic diagnostic apparatus according to any one of [7] to [10], in which the mammary gland region extraction unit extracts the mammary gland region using a trained model that has been trained through machine learning based on a plurality of training data each including the ultrasonic image in which the breast is imaged and the mammary gland region in the breast.
[13] The ultrasonic diagnostic apparatus according to any one of [1] to [12], in which the first category determination unit determines the category of the glandular tissue component based on a plurality of the ultrasonic images captured at a plurality of predetermined locations of the breast of the subject.
[14] The ultrasonic diagnostic apparatus according to any one of [1] to [13], in which the ultrasonic image is a three-dimensional ultrasonic image, and the first category determination unit determines the category of the glandular tissue component based on the three-dimensional ultrasonic image.
[15] A method of controlling an ultrasonic diagnostic apparatus, the method comprising: determining, based on an ultrasonic image including a mammary gland region in a breast of a subject, a category of a glandular tissue component in the breast; and outputting the determined category of the glandular tissue component.

According to the present invention, since the ultrasonic diagnostic apparatus comprises the first category determination unit that determines, based on the ultrasonic image including the mammary gland region in the breast of the subject, the category of the glandular tissue component in the breast, and the category output unit that outputs the category of the glandular tissue component determined by the first category determination unit, it is possible to consider the risk of cancer in more detail than the approach of only observing the mammary gland region in the related art.

### BRIEF DESCRIPTION OF THE DRAWINGS

Fig. 1 is a block diagram showing a configuration of an ultrasonic diagnostic apparatus according to Embodiment 1 of the present invention.
Fig. 2 is a block diagram showing an internal configuration of a transmission-and-reception circuit according to Embodiment 1.
Fig. 3 is a block diagram showing an internal configuration of an image generation unit according to Embodiment 1.
Fig. 4 is a diagram showing an ultrasonic image obtained by imaging a mammary gland region of a subject.
Fig. 5 is a diagram showing a display example of a category of a glandular tissue component.
Fig. 6 is a flowchart showing an operation according to Embodiment 1.
Fig. 7 is a flowchart showing a modification example of the operation according to Embodiment 1.
Fig. 8 is a block diagram showing an internal configuration of a first category determination unit according to Embodiment 2.
Fig. 9 is a block diagram showing an internal configuration of a first category determination unit according to Embodiment 3.
Fig. 10 is a diagram showing an ultrasonic image including a mammary gland region in which a GTC region is imaged.
Fig. 11 is a diagram showing a binarized image obtained by binarizing the mammary gland region by using a brightness threshold value.
Fig. 12 is a block diagram showing a configuration of an ultrasonic diagnostic apparatus according to Embodiment 4.
Fig. 13 is a diagram showing a configuration of a breast schematic diagram generated according to Embodiment 4.
Fig. 14 is a diagram showing a breast schematic diagram with a probe mark.
Fig. 15 is a flowchart showing an operation according to Embodiment 4.

### DESCRIPTION OF THE PREFERRED EMBODIMENTS

Hereinafter, embodiments of the invention will be described with reference to the accompanying drawings.

The following configuration requirements are described based on a representative embodiment of the present invention, but the present invention is not limited to such an embodiment.

It should be noted that, in the present specification, a numerical range represented by "to" means a range including numerical values described before and after "to", both ends inclusive, as a lower limit value and an upper limit value.

In the present specification, "same" and "identical" include an error range that is generally allowed in the technical field.

### [Embodiment 1]

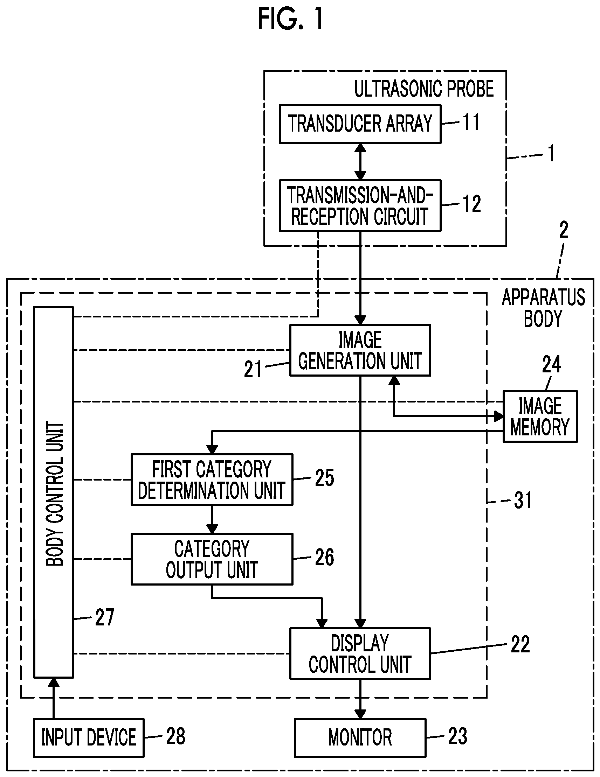

Fig. 1 shows a configuration of an ultrasonic diagnostic apparatus according to Embodiment 1 of the present invention. The ultrasonic diagnostic apparatus comprises an ultrasonic probe 1 and an apparatus body 2. The ultrasonic probe 1 and the apparatus body 2 are connected to each other in a wired manner via a cable (not shown).

The ultrasonic probe 1 includes a transducer array 11 and a transmission-and-reception circuit 12 connected to the transducer array 11.

The apparatus body 2 includes an image generation unit 21 connected to the transmission-and-reception circuit 12 of the ultrasonic probe 1, a display control unit 22 and a monitor 23 are sequentially connected to the image generation unit 21, and an image memory 24 is connected to the image generation unit 21. Further, a first category determination unit 25 and a category output unit 26 are sequentially connected to the image memory 24. The category output unit 26 is connected to the display control unit 22.

In addition, a body control unit 27 is connected to the transmission-and-reception circuit 12, the image generation unit 21, the display control unit 22, the image memory 24, the first category determination unit 25, and the category output unit 26. An input device 28 is connected to the body control unit 27. In addition, the image generation unit 21, the display control unit 22, the first category determination unit 25, the category output unit 26, and the body control unit 27 constitute a processor 31 for the apparatus body 2.

The transducer array 11 of the ultrasonic probe 1 includes a plurality of ultrasonic transducers arranged in a one-dimensional or two-dimensional manner. Each of these transducers transmits an ultrasonic wave in response to a drive signal supplied from the transmission-and-reception circuit 12, receives a reflected wave from a subject, and outputs an analog reception signal. Each transducer is configured by, for example, forming electrodes on both ends of a piezoelectric body consisting of a piezoelectric single crystal represented by lead zirconate titanate (PZT), a polymeric piezoelectric element represented by poly vinylidene di fluoride (PVDF), or a piezoelectric single crystal represented by a lead magnesium niobate-lead titanate (PMN-PT) solid solution.

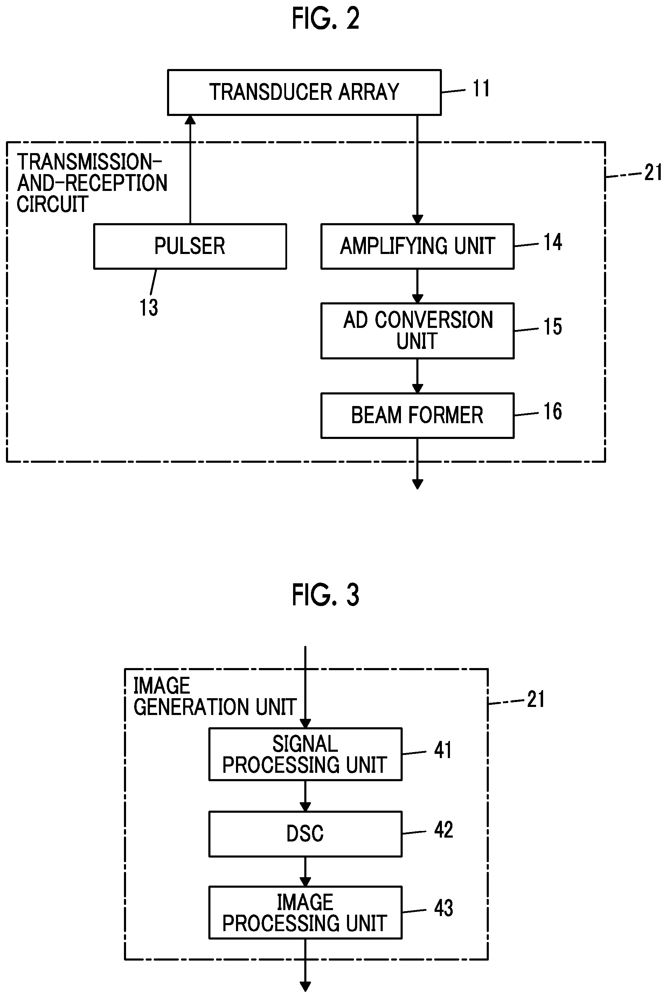

The transmission-and-reception circuit 12 transmits the ultrasonic wave from the transducer array 11 and generates a sound ray signal based on the reception signal acquired by the transducer array 11, under the control of the body control unit 27. As shown in Fig. 2, the transmission-and-reception circuit 12 includes a pulser 13 connected to the transducer array 11, and an amplifying unit 14, an analog-to-digital (AD) conversion unit 15, and a beam former 16 which are sequentially connected in series to the transducer array 11.

The pulser 13 includes, for example, a plurality of pulse generators, adjusts a delay amount of each drive signal based on a transmission delay pattern selected in accordance with a control signal from the body control unit 27 such that ultrasonic waves to be transmitted from the plurality of transducers of the transducer array 11 form a ultrasonic beam, and supplies the drive signal of which the delay amount has been adjusted, to the plurality of transducers. In this manner, in a case in which a pulsed or continuous wave voltage is applied to the electrodes of the transducers of the transducer array 11, the piezoelectric body expands and contracts to generate a pulsed or continuous wave ultrasonic wave from each transducer, and the ultrasonic beam is formed from the combined wave of these ultrasonic waves.

The transmitted ultrasonic beam is reflected by a target, for example, a part of the subject, and an ultrasonic echo propagates toward the transducer array 11 of the ultrasonic probe 1. The ultrasonic echo propagating toward the transducer array 11 in this manner is received by each of the transducers constituting the transducer array 11. In this case, each transducer constituting the transducer array 11 expands and contracts by receiving the propagating ultrasonic echo to generate the reception signal that is an electric signal, and outputs the reception signal to the amplifying unit 14.

The amplifying unit 14 amplifies the signal input from each of the transducers constituting the transducer array 11 and transmits the amplified signal to the AD conversion unit 15. The AD conversion unit 15 converts the signal transmitted from the amplifying unit 14 into digital reception data, and transmits the reception data to the beam former 16. The beam former 16 performs so-called reception focus processing by giving and adding delay with respect to each reception data converted by the AD conversion unit 15, in accordance with a sound velocity or a sound velocity distribution set based on a reception delay pattern selected according to a control signal from the body control unit 27. Through the reception focus processing, a sound ray signal is acquired in which each piece of the reception data converted by the AD conversion unit 15 is phased and added and the focus of the ultrasonic echo is narrowed.

As shown in Fig. 3, the image generation unit 21 of the apparatus body 2 has a configuration in which a signal processing unit 41, a digital scan converter (DSC) 42, and an image processing unit 43 are sequentially connected in series.

The signal processing unit 41 performs, on the sound ray signal transmitted from the transmission-and-reception circuit 12 of the ultrasonic probe 1, correction of attenuation caused by a distance in accordance with a depth of a reflection position of the ultrasonic wave and then performs envelope detection processing, and thereby generates an ultrasonic image signal (B-mode image signal), which is tomographic image information related to tissues in the subject.

The DSC 42 converts (raster-converts) the ultrasonic image signal generated by the signal processing unit 41 into an image signal in accordance with a normal television signal scanning method.

The image processing unit 43 performs various types of necessary image processing, such as gradation processing, on the ultrasonic image signal input from the DSC 42, and then outputs the signal representing the ultrasonic image to the display control unit 22 and the image memory 24. The signal representing the ultrasonic image generated by the image generation unit 21 in this way will be simply referred to as the ultrasonic image. It should be noted that the image generation unit 21 can also output the ultrasonic image signal before being processed by the DSC 42 or the ultrasonic image signal immediately after being processed by the DSC 42 to the image memory 24. In this case, the image generation unit 21 can generate the ultrasonic image by reading out these signals from the image memory 24 and performing processing using the DSC 42 or the image processing unit 43.

The display control unit 22 performs predetermined processing on the ultrasonic image transmitted from the image generation unit 21 under the control of the body control unit 27, and displays the ultrasonic image on the monitor 23.

The monitor 23 displays the ultrasonic image under the control of the display control unit 22, and includes, for example, a display device such as a liquid crystal display (LCD) or an organic electroluminescence display (organic EL display).

The image memory 24 is a memory that stores the ultrasonic image generated by the image generation unit 21 under the control of the body control unit 27. For example, the image memory 24 can store a plurality of frames of ultrasonic images generated by the image generation unit 21 in correspondence with diagnosis on a mammary gland region of a breast of the subject.

As the image memory 24, a recording medium such as a flash memory, a hard disc drive (HDD), a solid state drive (SSD), a flexible disc (FD), a magneto-optical disc (MO disc), a magnetic tape (MT), a random access memory (RAM), a compact disc (CD), a digital versatile disc (DVD), a secure digital card (SD card), or a universal serial bus memory (USB memory), can be used.

The first category determination unit 25 determines, based on a ultrasonic image including the mammary gland region in the breast of the subject, a category of a glandular tissue component (GTC) in the breast.

Here, the mammary gland region includes a GTC region including a mammary duct, a lobule, and perilobular stroma. In the mammary gland region, a space between the perilobular stroma is filled with edematous stroma. In general, it is known that the lobule atrophies with age, but there are research results that a risk of breast cancer is high in patients in whom the lobule does not atrophy, as disclosed in, for example, "Su Hyun Lee et al., "Glandular Tissue Component and Breast Cancer Risk in Mammographically Dense Breasts at Screening Breast US", Radiology, Volume 301, October 1, 2021". The category of the GTC represents a degree of progress of the atrophy of the lobule, and can be used as a material for determining the risk of breast cancer.

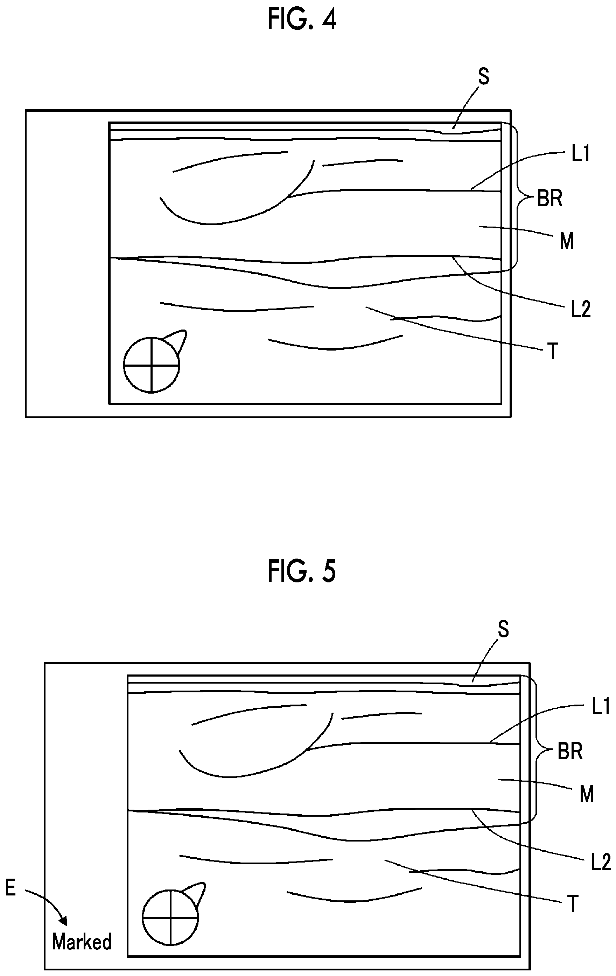

Fig. 4 shows an example of an ultrasonic image in which the breast of the subject is imaged. The ultrasonic image is a tomographic image captured by bringing a distal end of the ultrasonic probe 1 into contact with the breast of the subject, in which a skin S of the subject is shown in an upper end of the ultrasonic image representing a shallowest portion, and a pectoralis major T is shown in a lower portion of the ultrasonic image representing a deeper portion. A breast region BR is located in a region between the skin S and the pectoralis major T.

The first category determination unit 25 can determine the category of the GTC using, for example, a trained model that has been trained through machine learning based on a plurality of training data each including the ultrasonic image in which the breast is imaged and the category of the GTC in the breast as shown in Fig. 4. The association between the ultrasonic image and the category of the GTC in the training data can be performed by an expert such as a skilled doctor.

The first category determination unit 25 can output, as the category of the GTC, any one of a plurality of predetermined categories, for example, any one of four categories of Minimal, Mild, Moderate, or Marked. Mild indicates that the atrophy of the lobule is not more advanced than that in Minimal, Moderate indicates that the atrophy of the lobule is not more advanced than that in Mild, and Marked indicates that the atrophy of the lobule is not more advanced than that in Moderate. Further, the first category determination unit 25 can also determine, for example, any one of two categories of Low or High as the category of the GTC.

The category output unit 26 outputs the category of the GTC determined by the first category determination unit 25, and transmits the output category to the display control unit 22. The display control unit 22 can display the category of the GTC on the monitor 23 by, for example, a message E shown in Fig. 5. In the example of Fig. 5, the message E representing "Marked" is displayed. A user, such as a doctor, can easily understand the category of the GTC of the subject by confirming the message E, and can easily perform risk management regarding breast cancer on the subject by considering the risk of cancer in the mammary gland region in detail.

The body control unit 27 controls each unit of the apparatus body 2 and the transmission-and-reception circuit 12 of the ultrasonic probe 1 based on a control program or the like, which is stored in advance.

In addition, although not shown, a body-side storage unit is connected to the body control unit 27. The body-side storage unit stores the control program or the like. Further, as the body-side storage unit, for example, a flash memory, a RAM, an SD card, an SSD, or the like can be used.

The input device 28 is a device that allows the user to perform an input operation, and includes, for example, devices such as a keyboard, a mouse, a trackball, a touch pad, and a touch sensor provided in a superimposed manner on the monitor 23.

It should be noted that, although the processor 31 including the image generation unit 21, the display control unit 22, the first category determination unit 25, the category output unit 26, and the body control unit 27 is configured by a central processing unit (CPU) and a control program causing the CPU to perform various types of processing, the processor 31 may be configured by a field programmable gate array (FPGA), a digital signal processor (DSP), an application specific integrated circuit (ASIC), a graphics processing unit (GPU), or other integrated circuits (ICs), or may be configured by a combination thereof.

In addition, the image generation unit 21, the display control unit 22, the first category determination unit 25, the category output unit 26, and the body control unit 27 of the processor 31 can also be configured by being integrated partially or entirely into one CPU or the like.

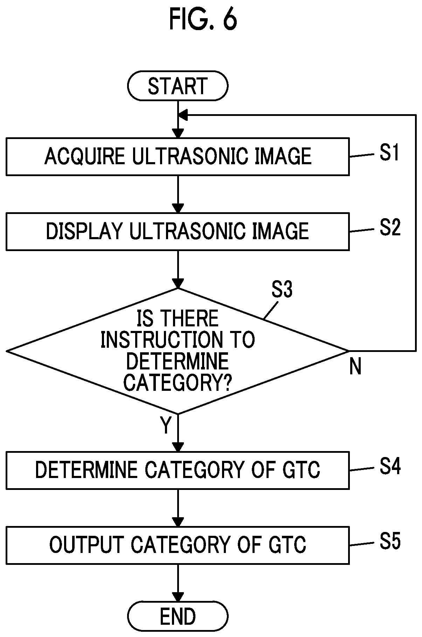

Next, an operation of the ultrasonic diagnostic apparatus according to Embodiment 1 will be described with reference to a flowchart shown in Fig. 6.

First, in step S1, the breast of the subject is imaged by using the ultrasonic probe 1, and the ultrasonic image is acquired. In this case, under the control of the body control unit 27, the transmission and reception of the ultrasonic waves from the plurality of transducers of the transducer array 11 are started in accordance with the drive signal from the pulser 13 of the transmission-and-reception circuit 12 of the ultrasonic probe 1, the ultrasonic echo from the inside of the breast of the subject is received by the plurality of transducers of the transducer array 11, the reception signal which is an analog signal is output to the amplifying unit 14 and is amplified by the amplifying unit 14, and the amplified reception signal is AD-converted by the AD conversion unit 15 to acquire the reception data.

The reception focus processing is performed on the reception data by the beam former 16, and the sound ray signal generated by the reception focus processing is transmitted to the image generation unit 21 of the apparatus body 2, and as a result, the ultrasonic image representing the tomographic image information of the breast of the subject is generated by the image generation unit 21. In this case, the signal processing unit 41 of the image generation unit 21 performs the correction of the attenuation in accordance with the depth of the reflection position of the ultrasonic wave and the envelope detection processing on the sound ray signal, the DSC 42 performs the conversion into the image signal in accordance with the normal television signal scanning method, and the image processing unit 43 performs various types of necessary image processing such as gradation processing.

Next, in step S2, the ultrasonic image generated by the image generation unit 21 is displayed on the monitor 23 via the display control unit 22, and is stored in the image memory 24.

It should be noted that, in a case in which the ultrasonic image is acquired, under the control of the body control unit 27, the transmission intensity of the ultrasonic wave and the depth range of the ultrasonic image displayed on the monitor 23 are adjusted such that the entire breast of the subject, that is, for example, a deep portion between a skin S and a pectoralis major T of the subject shown in Fig. 4, fits within a screen.

In next step S3, the body control unit 27 determines whether or not there is an instruction from the user to determine the category of the GTC. The body control unit 27 can determine that there is the instruction to determine the category of the GTC, for example, in a case in which the instruction to determine the category of the GTC is input from the user via the input device 28. In addition, for example, in a case in which the instruction to determine the category of the GTC is not input from the user via the input device 28, the body control unit 27 can determine that there is no instruction to determine the category of the GTC.

In a case in which it is determined in step S3 that there is no instruction to determine the category of the GTC, the processing returns to step S1, and a new ultrasonic image is acquired. In a case in which the ultrasonic image is newly acquired in this manner, the ultrasonic image is displayed on the monitor 23 in step S2, and the ultrasonic image is stored in the image memory 24. In this manner, the processing of step S1 to step S3 is repeated as long as it is determined that there is no instruction to determine the category of the GTC in step S3.

In the repetition of steps S1 to S3, the user adjusts the position of the ultrasonic probe 1 on the body surface of the subject while confirming the ultrasonic image displayed on the monitor 23. In a case in which it is determined in step S3 that there is the instruction to determine the category of the GTC, the processing proceeds to step S4.

In step S4, the first category determination unit 25 determines the category of the GTC in the breast of the subject based on the ultrasonic image stored in the image memory 24 in previous step S2. In this case, the first category determination unit 25 can determine the category of the GTC using, for example, a trained model that has been trained through machine learning based on a plurality of training data each including, for example, an ultrasonic image in which the breast is imaged and the category of the GTC in the breast. The first category determination unit 25 can determine, as the category of the GTC, for example, any one of the plurality of categories, such as any one of four categories of Minimal, Mild, Moderate, or Marked, or any one of two categories of Low or High.

In next step S5, the category output unit 26 outputs the category of the GTC determined in step S4. The category of the GTC output here is transmitted to the display control unit 22 and is displayed on the monitor 23 by, for example, the message E shown in Fig. 5. As a result, the user such as the doctor can easily understand the degree of progress of the atrophy of the lobule in the breast of the subject, and can easily and intuitively perform the risk management regarding breast cancer on the subject.

In a case in which the processing of step S5 is completed in this manner, the operation of the ultrasonic diagnostic apparatus according to the flowchart of Fig. 6 is completed.

As described above, with the ultrasonic diagnostic apparatus according to Embodiment 1, the first category determination unit 25 determines the category of the GTC in the breast based on the ultrasonic image including the mammary gland region in the breast of the subject, and the category output unit 26 outputs the category of the GTC, so that the user can easily understand the degree of progress of the atrophy of the lobule in the breast of the subject, and thereby easily performing the risk management for breast cancer of the subject and considering the risk of cancer in the mammary gland region in detail.

It should be noted that a case has been described in which the transmission-and-reception circuit 12 is provided in the ultrasonic probe 1, but the transmission-and-reception circuit 12 may be provided in the apparatus body 2.

Further, a case has been described in which the image generation unit 21 is provided in the apparatus body 2, but the image generation unit 21 may be provided in the ultrasonic probe 1.

In addition, the apparatus body 2 may be a so-called stationary type, a portable type that is easy to carry, or a so-called handheld type that is configured by, for example, a smartphone or a tablet type computer. As described above, the type of the device constituting the apparatus body 2 is not particularly limited.

In addition, a case has been described in which the ultrasonic probe 1 and the apparatus body 2 are connected to each other in a wired manner, but the ultrasonic probe 1 and the apparatus body 2 may be connected to each other in a wireless manner.

In Embodiment 1 described above, the ultrasonic image generated by the image generation unit 21 is a two-dimensional ultrasonic image, but the image generation unit 21 can also generate a three-dimensional ultrasonic image of the breast of the subject. A three-dimensional ultrasonic image may be generated by acquiring a plurality of two-dimensional ultrasonic images for a plurality of different tomographic planes by performing planar scanning with the ultrasonic probe 1, and then generating the three-dimensional ultrasonic image based on the plurality of two-dimensional ultrasonic images, or a three-dimensional probe may be used instead of the ultrasonic probe 1 to generate the three-dimensional ultrasonic image while the three-dimensional probe is stationary.

In addition, a case has been described in which the category of the GTC output by the category output unit 26 is displayed on the monitor 23 as the message E as shown in Fig. 5, but the method of notifying the user of the category of the GTC is not limited to this. For example, by providing a speaker (not shown) in the ultrasonic diagnostic apparatus, the category of the GTC can also be output as a voice through the speaker. In this case as well, the user can easily understand the degree of progress of the atrophy of the lobule in the breast of the subject.

The ultrasonic image can be represented as data in a so-called digital imaging and communications in medicine (DICOM) format, and can have a tag for storing the accessory information. In this case, the category of the GTC can be stored in the tag associated with the ultrasonic image. As a result, in a case in which the user such as the doctor confirms the ultrasonic image after the examination, the category of the GTC can also be confirmed.

In addition, the category of the GTC can also be stored in the image memory 24, for example, in a state of being superimposed on the corresponding ultrasonic image as text information.

In addition, the apparatus body 2 can include a report creation unit (not shown) that creates a report based on the category of the GTC output from the category output unit 26 and the corresponding ultrasonic image. The report includes at least the ultrasonic image and the category of the GTC corresponding to each other. The user such as the doctor can perform the risk management regarding breast cancer of the subject by confirming the category of the GTC with the report to consider the risk of cancer in the mammary gland region in detail.

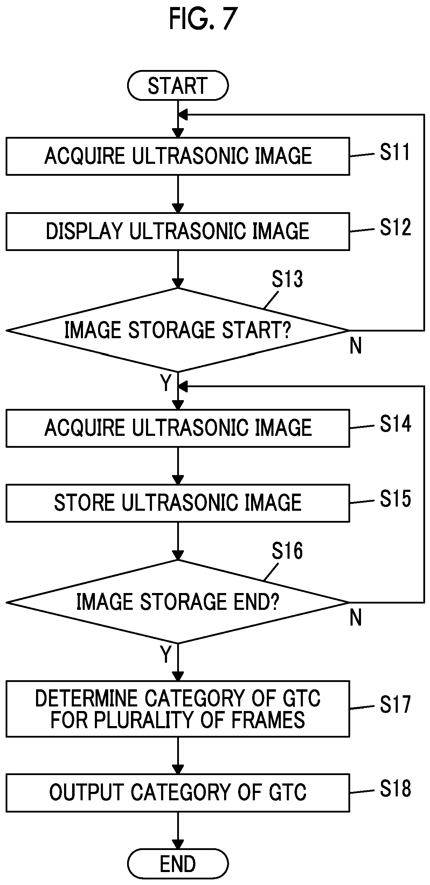

In addition, in the flowchart of Fig. 6, a case has been described in which the category of the GTC is determined based on the ultrasonic image of one frame, but the ultrasonic diagnostic apparatus according to Embodiment 1 can also determine the category of the GTC based on the plurality of frames of the ultrasonic images. This aspect will be described with reference to the flowchart of Fig. 7.

First, in step S11, as in step S1, the breast of the subject is imaged using the ultrasonic probe 1, and the ultrasonic image is acquired.

In step S12, the ultrasonic image acquired in step S11 is displayed on the monitor 23 as in step S2.

In step S13, the body control unit 27 determines whether or not to start the storage of the ultrasonic image. In this case, the body control unit 27 can determine to start the storage of the ultrasonic image, for example, in a case in which an instruction to start the storage of the ultrasonic image is input by the user via the input device 28. In addition, the body control unit 27 can determine not to start the storage of the ultrasonic image, for example, in a case in which the instruction to start the storage of the ultrasonic image is not input by the user via the input device 28.

In a case in which it is determined in step S13 not to start the storage of the ultrasonic image, the processing returns to step S11, a new ultrasonic image is acquired, and the ultrasonic image acquired in step S11 is displayed on the monitor 23 in next step S12. In this way, the processing of step S11 to step S13 is repeated as long as it is determined in step S13 not to start the storage of the ultrasonic image. In a case in which it is determined in step S13 to start the storage of the ultrasonic image, the processing proceeds to step S14.

In step S14, a new ultrasonic image is acquired as in step S11.

In step S15, the ultrasonic image acquired in step S14 is stored in the image memory 24.

In next step S16, the body control unit 27 determines whether or not to end the storage of the ultrasonic image. In this case, for example, in a case in which an instruction to end the storage of the ultrasonic image is input by the user via the input device 28, the body control unit 27 can determine to end the storage of the ultrasonic image. In addition, the body control unit 27 can determine to continue the storage of the ultrasonic image, for example, in a case in which the instruction to end the storage of the ultrasonic image is not input by the user via the input device 28.

In a case in which it is determined in step S16 to continue the storage of the ultrasonic image, the processing returns to step S14, a new ultrasonic image is acquired, and then the ultrasonic image is stored in the image memory 24 in step S15. In this way, the processing of step S14 to step S16 is repeated as long as it is determined in step S16 to continue the storage of the ultrasonic image. As a result, the plurality of frames of the ultrasonic images are stored in the image memory 24. In a case in which it is determined in step S16 to end the storage of the ultrasonic image, the processing proceeds to step S17.

In step S17, the first category determination unit 25 determines the category of the GTC for each of the plurality of frames of the ultrasonic images stored in the image memory 24 by repeating steps S14 to S16.

In step S18, the category output unit 26 calculates the final category of the GTC based on the plurality of categories of the GTC determined in step S17 based on the plurality of frames of the ultrasonic images, and outputs the calculated final category of the GTC.

The category output unit 26 can calculate, for example, the most frequent category among the plurality of categories of the GTC as the final category of the GTC. For example, in a case in which the category of Marked is determined most frequently among four categories of Minimal, Mild, Moderate, and Marked in step S17, the category output unit 26 can calculate the category of Marked as the final category of the GTC.

In a case in which the processing of step S18 is completed in this manner, the operation of the ultrasonic diagnostic apparatus according to the flowchart of Fig. 7 is completed.

It should be noted that, in the flowchart of Fig. 7, the category of the GTC is determined for each of the ultrasonic images after a plurality of desired frames of the ultrasonic images are stored in the image memory 24, but the category of the GTC of the acquired ultrasonic image can be determined immediately after the ultrasonic image is acquired, that is, immediately after step S14, and the determined category of the GTC can be stored in the image memory 24 in association with the ultrasonic image in step S15. In this case as well, in step S18, the final category of the GTC can be determined based on the plurality of categories calculated for the plurality of frames of the ultrasonic images.

### [Embodiment 2]

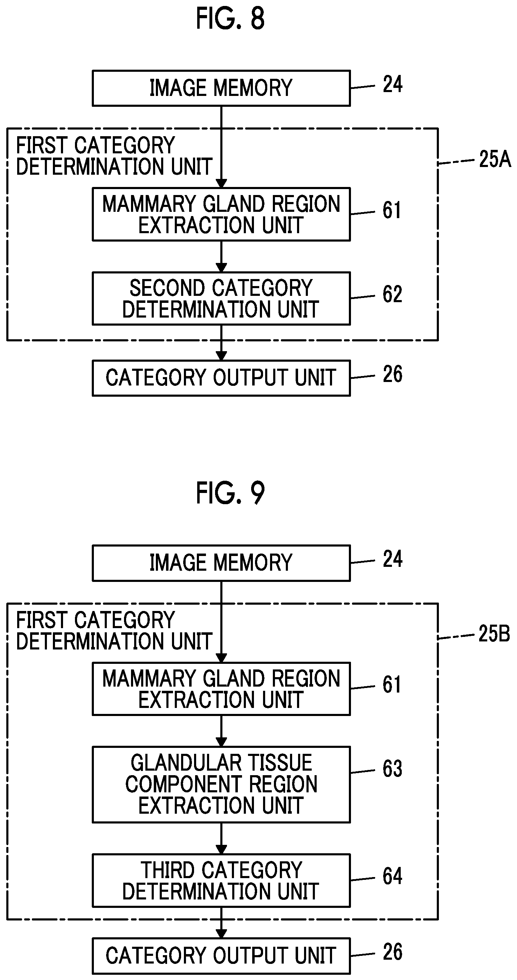

An ultrasonic diagnostic apparatus according to Embodiment 2 comprises a first category determination unit 25A shown in Fig. 8 instead of the first category determination unit 25 in the ultrasonic diagnostic apparatus according to Embodiment 1 shown in Fig. 1. The first category determination unit 25A has a configuration in which a mammary gland region extraction unit 61 and a second category determination unit 62 are connected in series.

The mammary gland region extraction unit 61 extracts the mammary gland region from the ultrasonic image including the mammary gland region in the breast of the subject. As shown in Fig. 4, the mammary gland region extraction unit 61 can recognize a front boundary line L1 located on a shallower side and a rear boundary line L2 located on a deeper side in the breast region BR, and can extract a deep region between the front boundary line L1 and the rear boundary line L2 as a mammary gland region M.

The mammary gland region extraction unit 61 can extract the mammary gland region by, for example, image-analyzing the ultrasonic image. In this case, the mammary gland region extraction unit 61 can extract the mammary gland region M by searching the inside of the ultrasonic image using, for example, so-called template matching, or an image analysis technique using a feature value such as adaptive boosting (Adaboost), support vector machine (SVM), or scale-invariant feature transform (SIFT).

In addition, the mammary gland region extraction unit 61 can also extract the mammary gland region M using, for example, a trained model that has been trained through machine learning based on a plurality of training data each including the ultrasonic image in which the breast is imaged and the mammary gland region M in the breast.

The second category determination unit 62 determines the category of the GTC in the breast based on the mammary gland region M extracted by the mammary gland region extraction unit 61. The second category determination unit 62 can determine the category of the GTC using, for example, a trained model that has been trained through machine learning based on a plurality of training data each including the ultrasonic image including the mammary gland region M in the breast, the mammary gland region M extracted by the mammary gland region extraction unit 61, and the category of the GTC in the breast. The mammary gland region M used in machine learning is represented in, for example, a form of so-called polygon coordinate information.

The category of the GTC determined by the first category determination unit 25A as described above is output by the category output unit 26. The category of the GTC output by the category output unit 26 is displayed on the monitor 23 by, for example, the message E as shown in Fig. 5.

As described above, even in a case in which the first category determination unit 25A is configured by the mammary gland region extraction unit 61 and the second category determination unit 62, in the same manner as the ultrasonic diagnostic apparatus according to Embodiment 1, the first category determination unit 25A determines the category of the GTC in the breast of the subject, and the category output unit 26 outputs the category of the GTC, so that the user can easily understand the degree of progress of the atrophy of the lobule in the breast of the subject, and can easily perform the risk management regarding breast cancer of the subject, by considering the risk of cancer in the mammary gland region M in detail.

### [Embodiment 3]

An ultrasonic diagnostic apparatus according to Embodiment 3 comprises a first category determination unit 25B shown in Fig. 9 instead of the first category determination unit 25 in the ultrasonic diagnostic apparatus according to Embodiment 1 shown in Fig. 1. The first category determination unit 25B has a configuration in which the mammary gland region extraction unit 61, a glandular tissue component region extraction unit 63, and a third category determination unit 64 are connected in series.

The glandular tissue component region extraction unit 63 extracts the GTC region including the mammary duct, the lobule, and the perilobular stroma in the mammary gland region M from the mammary gland region M extracted by the mammary gland region extraction unit 61. In the mammary gland region M, the space between the perilobular stroma is filled with the edematous stroma. Since the edematous stroma is rich in extracellular matrix and contains coexisting fat, in a case of observing the mammary gland region M using the ultrasonic image, the edematous stroma has a high echo level and appears bright. On the other hand, the mammary duct, the lobule, and the perilobular stroma constituting the GTC region have relatively low echo levels, and the brightness is lower than that of the edematous stroma.

The glandular tissue component region extraction unit 63 can extract the glandular tissue component region by, for example, image-analyzing the ultrasonic image in which the mammary gland region M is imaged. In this case, the glandular tissue component region extraction unit 63 can extract the GTC region by distinguishing between the GTC region and the edematous stroma in the mammary gland region M by, for example, binarizing the mammary gland region M of the ultrasonic image using a brightness threshold value Th. It should be noted that, in a case of binarizing the mammary gland region M of the ultrasonic image, it is preferable to normalize a histogram of the mammary gland region M.

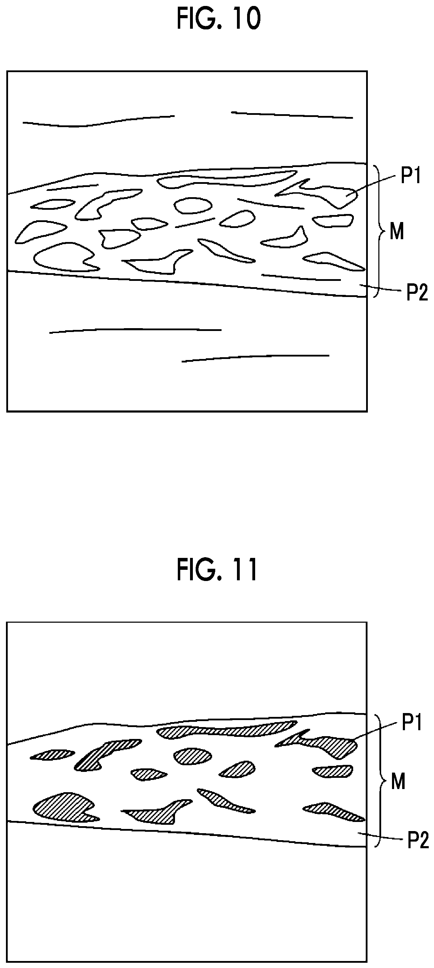

For example, as in the ultrasonic image shown in Fig. 10, in a case in which a GTC region R1 and a edematous region R2 filled with the edematous stroma coexist in the mammary gland region M, by binarizing the mammary gland region M using an appropriate brightness threshold value Th, a binarized image as shown in Fig. 11 is obtained.

In the binarized image of Fig. 11, pixels having brightness values lower than the brightness threshold value Th are represented in black (a shaded regions in Fig. 11) to form a black portion P1, and pixels having brightness values equal to or higher than the brightness threshold value Th are represented in white to form a white portion P2. The black portion P1 corresponds to the GTC region R1, and the white portion P2 corresponds to the edematous region R2. That is, the GTC region R1 can be extracted as the black portion P1 by binarizing the mammary gland region M.

The binarized image created by the glandular tissue component region extraction unit 63 can be displayed on the monitor 23, for example, through the display control unit 22.

It should be noted that a predetermined constant value can be used as the brightness threshold value Th.

In addition, the glandular tissue component region extraction unit 63 may perform edge detection on the GTC region R1 of the ultrasonic image by image analysis, and automatically calculate the brightness threshold value Th based on a change in brightness values of the detected edge portion, that is, a change in brightness values of a plurality of pixels from the inside to the outside of the GTC region R1. In this way, it is possible to automatically set the brightness threshold value Th suitable for the ultrasonic image as the image analysis target, and to acquire the binarized image suitable for the ultrasonic image.

In addition, the glandular tissue component region extraction unit 63 can also extract the GTC region R1, for example, using a trained model that has been trained through machine learning based on a plurality of training data each including the ultrasonic image in which the mammary gland region M and the GTC region R1 are imaged.

The third category determination unit 64 determines the category of the GTC in the breast of the subject based on the GTC region R1 extracted by the glandular tissue component region extraction unit 63.

The third category determination unit 64 can calculate, for example, a ratio of the GTC region R1 to the mammary gland region M, to determine the category of the GTC of the subject based on the calculated ratio (GTC region ratio) of the GTC region R1 to the mammary gland region M. In this case, the third category determination unit 64 can calculate the ratio based on, for example, the number of pixels occupied by the mammary gland region M and the number of pixels occupied by the GTC region R1 in the ultrasonic image. Specifically, the GTC region ratio is represented by a ratio of a sum of the number of pixels occupied by all the GTC regions R1 existing in the mammary gland region M to the number of pixels occupied by the entire mammary gland region M.

The third category determination unit 64 can determine the category of the GTC of the subject based on the GTC region ratio calculated as described above. For example, the third category determination unit 64 can determine the category of the GTC as Minimal in a case in which the GTC region ratio is less than 25%, determine the category of the GTC as Mild in a case in which the GTC region ratio is equal to or more than 25% and less than 50%, determine the category of the GTC as Moderate in a case in which the GTC region ratio is equal to or more than 50% and less than 75%, and determine the category of the GTC as Marked in a case in which the GTC region ratio is equal to or more than 75%. In addition, for example, the third category determination unit 64 can determine the category of the GTC as Low in a case in which the GTC region ratio is less than 50%, and can determine the category of the GTC as High in a case in which the GTC region ratio is equal to or more than 50%. As a result, the user need not confirm a numerical value of the GTC region ratio, for example, to determine the degree of progress of the atrophy of the lobule, and can intuitively understand the degree of progress of the atrophy of the lobule. It should be noted that the threshold value of the GTC region ratio in a case of determining the category of the GTC is not limited to these examples, and can be set to any value.

In addition, the third category determination unit 64 can also determine the category of the GTC using a trained model that has been trained through machine learning based on a plurality of training data each including the ultrasonic image in which the GTC region R1 is imaged and the category of the GTC in the breast.

The category of the GTC determined by the first category determination unit 25B as described above is output by the category output unit 26. The category of the GTC output by the category output unit 26 is displayed on the monitor 23 by, for example, the message E as shown in Fig. 5.

As described above, even in a case in which the first category determination unit 25B is configured by the mammary gland region extraction unit 61, the glandular tissue component region extraction unit 63, and the third category determination unit 64, in the same manner as the ultrasonic diagnostic apparatus according to Embodiment 1, the first category determination unit 25B determines the category of the GTC in the breast of the subject, and the category output unit 26 outputs the category of the GTC, so that the user can easily and intuitively understand the degree of progress of the atrophy of the lobule in the breast of the subject, and can easily perform the risk management regarding breast cancer for the subject.

It should be noted that, in Embodiment 3, as shown in the flowchart of Fig. 7, in a case in which the category of the GTC is determined based on the plurality of frames of the ultrasonic images, the third category determination unit 64 can calculate, for example, values of a plurality of GTC region ratios based on the plurality of frames of the ultrasonic images, calculate an average value or a median value of the calculated values of the plurality of GTC region ratios, and determine the final category of the GTC based on the calculated average value or median value. As a result, the accuracy of the category of the GTC can be improved.

### [Embodiment 4]

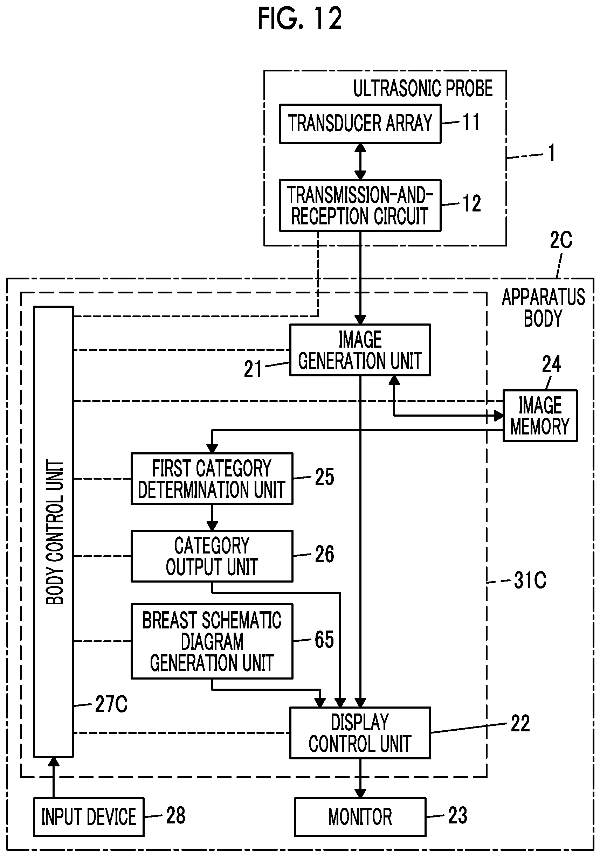

Fig. 12 shows a configuration of an ultrasonic diagnostic apparatus according to Embodiment 4. The ultrasonic diagnostic apparatus according to Embodiment 4 comprises an apparatus body 2C instead of the apparatus body 2 in the ultrasonic diagnostic apparatus according to Embodiment 1 shown in Fig. 1.

The apparatus body 2C further comprises a breast schematic diagram generation unit 65 and comprises a body control unit 27C instead of the body control unit 27, in the apparatus body 2 according to Embodiment 1. In the apparatus body 2C, the breast schematic diagram generation unit 65 is connected to the display control unit 22 and the body control unit 27C. In addition, the image generation unit 21, the display control unit 22, the first category determination unit 25, the category output unit 26, the body control unit 27C, and the breast schematic diagram generation unit 65 constitute a processor 31C for the apparatus body 2.

In one ultrasonic image obtained by bring the ultrasonic probe 1 to come into contact with one location of the breast of the subject and imaging the breast of the subject, normally, only a local tomographic plane of the breast can be seen, and thus, in the ultrasonic diagnostic apparatus according to Embodiment 4, the ultrasonic images are captured by using the ultrasonic probe 1 at the plurality of predetermined locations of the breast.

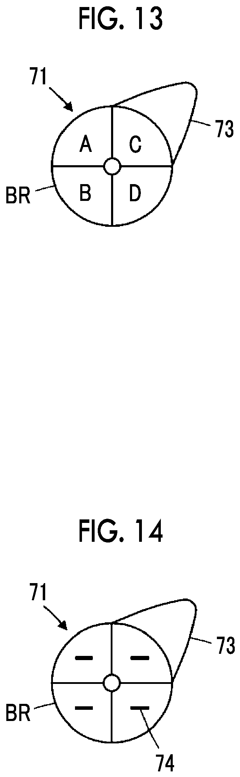

Therefore, the breast schematic diagram generation unit 65 generates, for example, a breast schematic diagram (also referred to as a schema or a body mark) 71 as shown in Fig. 13. The breast schematic diagram 71 shown in Fig. 13 schematically represents a left breast in a case of viewed from the front, and includes a circle-shaped breast region BR and a substantially triangular axillary region 73 representing an armpit and extending obliquely upward from the breast region BR. The breast region BR is divided into four regions of the breast: an inner upper region A, an inner lower region B, an outer upper region C, and an outer lower region D, and the axillary region 73 is connected to a left oblique upper portion of the outer upper region C.

It should be noted that, by horizontally inverting the breast schematic diagram 71 shown in Fig. 13, a breast schematic diagram schematically representing a right breast is obtained.

The breast schematic diagram generation unit 65 generates the breast schematic diagram 71 in which a plurality of predetermined locations for capturing the ultrasonic images by applying the ultrasonic probe 1 thereto are plotted with probe marks 74, as shown in Fig. 14, by using the inner upper region A, the inner lower region B, the outer upper region C, and the outer lower region D obtained by dividing the breast region BR into four regions, and displays the breast schematic diagram 71 on the monitor 23.

In the breast schematic diagram 71 of Fig. 14, the probe marks 74 are plotted in all the four regions obtained by dividing the breast region BR. It should be noted that the probe mark 74 is represented by a line segment having a predetermined length, and not only indicates a position of each of the plurality of locations to which the ultrasonic probe 1 is applied but also indicates a direction of the ultrasonic probe 1 applied each location by a direction of the line segment.

In the ultrasonic diagnostic apparatus according to Embodiment 4, the first category determination unit 25 determines the category of the GTC in the breast of the subject based on each of the plurality of ultrasonic images captured at the plurality of locations determined by the breast schematic diagram 71, and the category output unit 26 outputs the category of the GTC determined by the first category determination unit 25.

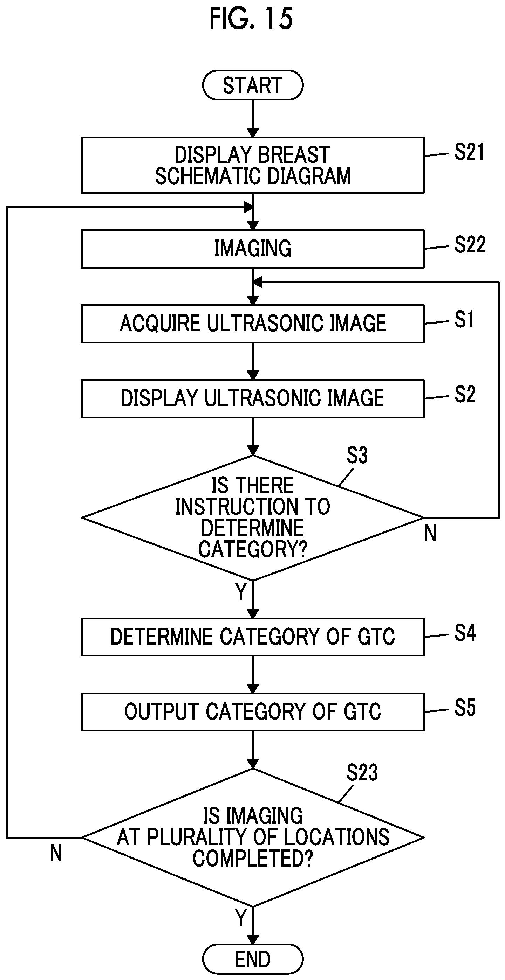

An operation of the ultrasonic diagnostic apparatus according to Embodiment 4 will be described with reference to a flowchart shown in Fig. 15.

First, in step S21, the breast schematic diagram 71 as shown in Fig. 14 is generated by the breast schematic diagram generation unit 65, and the breast schematic diagram 71 is displayed on the monitor 23 via the display control unit 22. In the breast schematic diagram 71 shown in Fig. 14, the probe marks 74 are plotted in all the four regions obtained by dividing the breast region BR.

In step S22, the user confirms the breast schematic diagram 71 displayed on the monitor 23, and captures the ultrasonic image in accordance with the probe mark 74 plotted in one region of the four regions. That is, the ultrasonic probe 1 is brought into contact with the breast of the subject in accordance with the position and the direction indicated by the probe mark 74, and in this state, the plurality of transducers of the transducer array 11 start the transmission and reception of the ultrasonic waves, and the ultrasonic echoes from the inside of the breast of the subject are received by the plurality of transducers of the transducer array 11.

Steps S1 to S5 following step S22 are the same as steps S1 to S5 of the flowchart in Embodiment 1 shown in Fig. 6. In a case in which it is determined that the ultrasonic image is acquired in step S1, that the ultrasonic image is displayed on the monitor 23 in step S2, and that there is the instruction to determine the category of the GTC in step S3, the first category determination unit 25 determines the category of the GTC in the breast of the subject based on the ultrasonic image in step S4, and the category output unit 26 outputs the category of the GTC in step S4.

Thereafter, in step S23, it is determined whether or not capturing of the ultrasonic images for a plurality of predetermined locations is completed. Here, only the imaging in accordance with a first probe mark 74 of the four probe marks 74 plotted on the breast schematic diagram 71 is completed, and the imaging in accordance with the remaining three probe marks 74 is not performed, so that it is determined that imaging at the plurality of locations is not yet completed, and the processing returns from step S23 to step S22.

In step S22, the imaging is performed in accordance with a second probe mark 74, and in next steps S1 to S5, the category of the GTC is determined and output based on the ultrasonic image acquired in accordance with the second probe mark 74, and then it is determined in step S23 whether or not the imaging is completed at a plurality of locations.

In the same manner, step S22, steps S1 to S5, and step S23 are repeated until the ultrasonic image is captured for all four probe marks 74 plotted on the breast schematic diagram 71.

In addition, in a case in which it is determined in step S23 that capturing of the ultrasonic images for all the four probe marks 74 is completed, a series of processing is completed.

As a result, the category of the GTC is determined and output for each of the four locations corresponding to the four probe marks 74 in the breast in the breast schematic diagram 71.

By determining the category of the GTC based on the plurality of ultrasonic images captured for the plurality of locations, the accuracy of estimating the risk of breast cancer in the mammary gland region M of the subject is improved, and it is possible to perform a more reliable diagnosis.

It should be noted that, in the breast schematic diagram 71 shown in Fig. 14, the probe marks 74 are plotted in all the four regions obtained by dividing the breast region BR, but the probe marks 74 may be plotted in two or more regions among the four regions instead of all the four regions. Further, the number of regions obtained by dividing the breast region BR is not limited to four, and for example, the breast region BR may be divided into two regions or eight regions.

Further, instead of plotting the probe marks 74 in the regions obtained by dividing the breast region BR, as shown in Fig. 13, the regions such as the inner upper region A, the inner lower region B, the outer upper region C, and the outer lower region D may be designated in the breast schematic diagram 71, and the user may capture the ultrasonic image at a certain position in the designated region.

The number of divided regions in the breast schematic diagram 71, the number of the probe marks 74 to be plotted, and the like may be automatically set by the breast schematic diagram generation unit 65 under the control of the body control unit 27C, or may be manually set by the user via the input device 28.

### Explanation of References

1: ultrasonic probe
2, 2C: apparatus body
11: transducer array
12: transmission-and-reception circuit
13: pulser
14: amplifying unit
15: AD conversion unit
16: beam former
21: image generation unit
22: display control unit
23: monitor
24: image memory
25, 25A, 25B: first category determination unit
26: category output unit
27, 27C: body control unit
28: input device
31, 31C: processor
41: signal processing unit
42: DSC
43: image processing unit
61: mammary gland region extraction unit
62: second category determination unit
63: glandular tissue component region extraction unit
64: third category determination unit
65: breast schematic diagram generation unit
BR: breast region
E: message
L1: front boundary line
L2: rear boundary line
P1: black portion
P2: white portion
R1: GTC region
R2: edematous region
S: skin
T: pectoralis major

## Claims

1. An ultrasonic diagnostic apparatus comprising:
a first category determination unit that determines, based on an ultrasonic image including a mammary gland region in a breast of a subject, a category of a glandular tissue component in the breast; and
a category output unit that outputs the category of the glandular tissue component determined by the first category determination unit.

2. The ultrasonic diagnostic apparatus according to claim 1,
wherein the first category determination unit determines the category of the glandular tissue component using a trained model that has been trained through machine learning based on a plurality of training data each including the ultrasonic image in which the breast is imaged and the category of the glandular tissue component in the breast.

3. The ultrasonic diagnostic apparatus according to claim 1,
wherein the first category determination unit includes
a mammary gland region extraction unit that extracts the mammary gland region from the ultrasonic image in which the breast of the subject is imaged, and
a second category determination unit that determines the category of the glandular tissue component in the breast based on the mammary gland region extracted by the mammary gland region extraction unit.

4. The ultrasonic diagnostic apparatus according to claim 3,
wherein the second category determination unit determines the category of the glandular tissue component using a trained model that has been trained through machine learning based on a plurality of training data each including the ultrasonic image including the mammary gland region in the breast, the mammary gland region extracted by the mammary gland region extraction unit, and the category of the glandular tissue component in the breast.

5. The ultrasonic diagnostic apparatus according to claim 3 or 4,
wherein the mammary gland region extraction unit extracts the mammary gland region by image-analyzing the ultrasonic image.

6. The ultrasonic diagnostic apparatus according to claim 3 or 4,
wherein the mammary gland region extraction unit extracts the mammary gland region using a trained model that has been trained through machine learning based on a plurality of training data each including the ultrasonic image in which the breast is imaged and the mammary gland region in the breast.

7. The ultrasonic diagnostic apparatus according to claim 1,
wherein the first category determination unit includes
a mammary gland region extraction unit that extracts the mammary gland region from the ultrasonic image in which the breast of the subject is imaged,
a glandular tissue component region extraction unit that extracts a glandular tissue component region including a mammary duct, a lobule, and perilobular stroma in the mammary gland region, from the mammary gland region extracted by the mammary gland region extraction unit, and
a third category determination unit that determines the category of the glandular tissue component in the breast based on the glandular tissue component region extracted by the glandular tissue component region extraction unit.

8. The ultrasonic diagnostic apparatus according to claim 7,
wherein the third category determination unit determines the category of the glandular tissue component using a trained model that has been trained through machine learning based on a plurality of training data each including the ultrasonic image in which the glandular tissue component region is imaged and the category of the glandular tissue component in the breast.

9. The ultrasonic diagnostic apparatus according to claim 7,
wherein the third category determination unit calculates a ratio of the glandular tissue component region to the mammary gland region, to determine the category of the glandular tissue component of the subject based on the calculated ratio of the glandular tissue component region to the mammary gland region.

10. The ultrasonic diagnostic apparatus according to any one of claims 7 to 9,
wherein the glandular tissue component region extraction unit extracts the glandular tissue component region by image-analyzing the ultrasonic image in which the mammary gland region is imaged.

11. The ultrasonic diagnostic apparatus according to any one of claims 7 to 9,
wherein the mammary gland region extraction unit extracts the mammary gland region by image-analyzing the ultrasonic image.

12. The ultrasonic diagnostic apparatus according to any one of claims 7 to 9,
wherein the mammary gland region extraction unit extracts the mammary gland region using a trained model that has been trained through machine learning based on a plurality of training data each including the ultrasonic image in which the breast is imaged and the mammary gland region in the breast.

13. The ultrasonic diagnostic apparatus according to claim 1,
wherein the first category determination unit determines the category of the glandular tissue component based on a plurality of the ultrasonic images captured at a plurality of predetermined locations of the breast of the subject.

14. The ultrasonic diagnostic apparatus according to claim 1,
wherein the ultrasonic image is a three-dimensional ultrasonic image, and
the first category determination unit determines the category of the glandular tissue component based on the three-dimensional ultrasonic image.

15. A method of controlling an ultrasonic diagnostic apparatus, the method comprising:
determining, based on an ultrasonic image including a mammary gland region in a breast of a subject, a category of a glandular tissue component in the breast; and
outputting the determined category of the glandular tissue component.
